# EUROPEAN PATENT APPLICATION

(11) **EP 1 215 498 A1**
(43) Date of publication of application: **19.06.2002**
(21) Application number: 00961205.2
(22) Date of filing: 22.09.2000
(51) Int. Cl.: G01N 33/566, G01N 33/58, G01N 33/53, C12Q 1/68, C12N 15/00

(54) **HYBRIDIZATION SELF-RECOGNITION TYPE PROBE**

(30) Priority: 22.09.1999 JP 26874599
(71) Applicant: WAKUNAGA PHARMACEUTICAL CO., LTD., Osaka-shi, Osaka 532-0003 (JP)
(72) Inventor: Yamane, Akio Wakunaga Pharmaceutical Co., Ltd., Takata-gun, Hiroshima 739-1105 (JP)
(74) Representative: Warcoin, Jacques
(86) International application number: JP0006524
(87) International publication number: WO0122089

(57) **Abstract**

An objective of the present invention is to provide a probe for detecting a nucleic acid, in which a gene can be detected by a simple operation in a short time with high sensitivity. The probe according to the present invention comprises a nucleic acid carrying a labeling substance that releases energy and an energy-absorbing substance capable of absorbing energy released from the labeling substance, wherein energy transfer from the labeling substance to the energy-absorbing substance is intercepted by the hybridization of the probe with the target nucleic acid.

SEQUENCE LISTING

<110> Wakunaga Pharmaceutical Co., Ltd.

<120> Hybridization self-recognition probe

<130> 127533PX

<140>
<141>

<150> 268745/1999
<151> 1999-09-22

<160> 4

<170> PatentIn Ver. 2.1

<210> 1
<211> 17
<212> DNA
<213> Artificial Sequence

<220>
<223> Description of Artificial Sequence:Hybridization
      probe

<400> 1
gcaacaggcc gacaacg                                                17

<210> 2
<211> 17
<212> DNA
<213> Artificial Sequence

<220>
<223> Description of Artificial Sequence:Hybridization
      probe

<400> 2
acgcacaaaa ccaagca                                                    17

<210> 3
<211> 17
<212> DNA
<213> Artificial Sequence

<220>
<223> Description of Artificial Sequence:hybridization
      probe

<400> 3
cgttgtcggc ctgttgc                                                    17

<210> 4

<211> 17
<212> DNA
<213> Artificial Sequence

<220>
<223> Description of Artificial Sequence:Hybridization
      probe

<400> 4
tgcttggttt tgtgcgt                                                17

## Description

### BACKGROUND OF THE INVENTION

### Field of the Invention

The present invention relates to a self-recognition probe for use in detecting a nucleic acid. In particular, the present invention relates to a probe in which energy release (for example, fluorescence) from a labeling substance is controlled by an energy-absorbing substance.

### Description of the Related Art

The great progress in medical genetics has been made in recent years, and decoding the entire nucleotide sequences of the human genes will soon be completed. Further, data of relationship between genetic polymorphisms and diseases have been accumulated day by day, and analysis of polymorphisms in the entire region of human genes is in progress. On the other hand, genetic tests for viruses and bacteria, which cause human diseases, have become available for practical use and contributed to medical treatment. Under these circumstances, expectation in genetic testing technology is high, and further simplification and cost reduction are needed.

Conventionally, methods for detecting a specific gene by using probes have been available. Generally, in these methods, a sample containing a nucleic acid to be detected is immobilized on a solid phase, a double-stranded chain is formed by hybridization with a nucleic acid complementary to this target (the nucleic acid complementary to the nucleic acid to be detected is referred to as a "probe" hereinafter), into which a labeling substance is introduced, and the solid phase is washed, after which the labeling substance in the nucleic acid trapped in the solid phase is detected to examine the presence or absence of the target nucleic acid in the sample. Further, in alternative detection methods, a sample nucleic acid is labeled and then allowed to form a duplex with a probe immobilized on a solid phase. These methods are well established and reliable; however, they require an excessive amount of sample, probe, reagent for detection, or the like, and the washing process is laborious, which partly impedes dissemination of the genetic testing.

In order to solve these problems, various methods have been examined; all of the methods examined involve so-called homogeneous hybridization that does not require solid-liquid separation. An example of such methods is a hybridization protection method using an acridinium ester (Arnold et al., Clin. Chem., 35, 1588-1594, 1988). In this method, an acridinium ester is introduced into a probe, and the system utilizes the fact that the probe is more resistant to alkaline hydrolysis when a duplex is formed than when a duplex is not formed. Namely, alkaline hydrolysis is carried out after hybridization, and the acridinium ester remained intact is detected by chemiluminescence. This method has an advantage that solid-liquid separation is not required and thus practically used in various areas. However, this method still requires treatment such as hydrolysis after hybridization, the acridinium ester is decomposed at a high temperature, and chemiluminescence occurs only once, which impedes further application of this method. On the other hand, methods with the use of energy transfer have been developed as a typical method for homogeneous hybridization (Kricka L.J., Nonisotopic DNA Probe Techniques, pp. 311-352, Academic Press, Inc., 1992). A representative of such methods involves the use of two probes, in which one label is introduced near the 5' end of one probe and the other label is introduced near the 3' end of the other probe. This method utilizes the fact that these two kinds of labels can come close by hybridization only when a nucleic acid complementary to both probes is present, which generates energy transfer between these two kinds of labels. In any of these methods, either fluorescence is quenched in the presence of the target gene or fluorescence is emitted as a result of the absorption of fluorescence on one label by the other fluorescent substance, which generates various problems such as background effect.

In order to solve these problems, new methods with the use of energy transfer have been developed. One of such methods is called the TaqMan™ (Livak, K.J. et al., PCR Methods Appl., 4, 357-362, 1995), which involves the use of two fluorescent labels introduced into an oligonucleotide, one making a donor and the other making an acceptor. The method utilizes the fact that this oligonucleotide is decomposed by exonuclease activity of DNA polymerase in the direction from 5' to 3', only when a duplex is formed with a complementary sequence. Namely, when these two kinds of fluorescent labels are present on the same oligonucleotide, they are close enough to cause energy transfer so that fluorescence of one fluorescent label is absorbed by the other fluorescent label and thus no fluorescence from the former label is observed. However, when the oligonucleotide is decomposed by exonuclease activity of DNA polymerase in the direction from 5' to 3', the two kinds of labels are present on separate molecules so that no energy transfer occurs, and thus fluorescence is observed. This method is widely used in combination with the polymerase chain reaction. However, it is difficult to detect a single base alteration by this method.

Another usable method is the Molecular Beacon method (Kramer F.R. et la., Nature Biotechnology, 49-53 1988). In this method, a fluorescent acceptor and a fluorescent donor are introduced into both termini of an oligonucleotide, and extra sequences other than a sequence complementary to a target are further added to both termini; a hairpin structure is formed with the extra sequences when no target is present. Namely, the hairpin structure formation brings the two kinds of labels close enough to produce energy transfer. On the other hand, when a target complementary to this oligonucleotide is present, a duplex with the target is formed, the hairpin structure is disrupted, and thus no energy transfer occurs. In other words, this method utilizes the fact that fluorescence of one fluorescent substance is absorbed by the other fluorescent substance in the absence of the target, while such quenching does not occur in the presence of the target.

Further, another method, which does not utilize energy transfer between two labels, has been developed, in which a substance that emits fluorescence upon binding to a double-stranded nucleic acid is introduced into an oligonucleotide (Ishiguro, T. et al., Nucleic Acids Res., 24, 4992-4997, 1996). However, this method may have problems such as an increased background.

Further, Sawai et al. (J. Chem. Soc., Chem. Commun., 1337-1378, 1994) have introduced a substance, in which fluorescein and acridine, an intercalator, are bonded via a spacer, into the 5' end of an oligonucleotide, and observed a mode of double-strand formation reaction of the oligonucleotide. Namely, when this oligonucleotide is present as a single-stranded form, fluorescent resonance energy transfer from acridine to fluorescein occurs upon radiation at 340 nm that is the absorption wavelength for acridine, and thus fluorescence from fluorescein is observed. On the other hand, when this oligonucleotide forms a double-stranded chain with another nucleic acid, the acridine moiety intercalates to the douplex so that fluorescent resonance energy transfer to fluorescein cannot efficiently occur upon radiation at 340 nm, and thus fluorescence from fluorescein cannot be detected. This method is novel in terms of the simultaneous introduction of a fluorescent substance and an intercalator into the terminus of an oligonucleotide. However, generally, background is often increased in a method, in which the first fluorescent substance is excited, the second fluorescent substance is excited by the resulting fluorescence, and the fluorescence emitted from the second fluorescent substance is measured, since the light used to excite the first fluorescent substance does also excite the second fluorescent substance to some degree. Further, this method is not suitable for general diagnosis because fluorescence is emitted in the absence of a complementary chain and fluorescence is diminished in the presence of a complementary chain.

### SUMMARY OF THE INVENTION

An objective of the present invention is to provide a probe for detecting a nucleic acid and a method for detecting a nucleic acid, in which a gene can be detected by a simple operation in a short time with high sensitivity without solid-liquid separation.

Another objective of the present invention is to provide a solid phase carrier for detecting a nucleic acid in which the target nucleic acid can be detected without labeling a sample.

A probe according to the present invention comprises a nucleic acid carrying a labeling substance that releases energy and an energy-absorbing substance capable of absorbing the energy released from the labeling substance, wherein energy transfer from the labeling substance to the energy-absorbing substance is intercepted by the hybridization of the probe with a target nucleic acid.

A method for detecting nucleic acid according to the present invention comprises the steps of contacting the abovementioned probe with a nucleic acid sample and then measuring energy released from the labeling substance.

A solid phase carrier for detecting a nucleic acid according to the present invention is a carrier on which the abovementioned probe is immobilized.

### BRIEF DESCRIPTION OF THE DRAWINGS

Figure 1 shows schematic diagrams of the probe according to the present invention. The "Single Stranded" illustrates the state where the probe is not hybridized with a target nucleic acid. The "Double Stranded" illustrates the state where the probe is hybridized with a target nucleic acid. F is a labeling substance such as fluorescein, and P is a photo-absorbing substance such as pyrene.
Figure 2 shows embodied structures of modified parts of oligonucleotides (EFN1-F and EFN2-F) which carry amino group and fluorescein. EFN3-F and EFN4-F have the same structures as EFN1-F and EFN2-F except for the bases.
Figure 3 shows embodied structures of modified parts of oligonucleotides (EFN1-FP and EFN2-FP) which carry pyrene and fluorescein. EFN3-FP and EFN4-FP have the same structure as EFN1-FP and EFN2-FP except for the bases.

### DETAILED DESCRIPTION OF THE INVENTION

The term "labeling substance" as used herein refers to any substance that becomes an excited state by receiving photochemical or chemical energy and releases energy upon returning from the excited state to the original state. Examples of the substance to be photochemically excited include fluorescent substances such as fluorescein and tetramethylrhodamine. Further examples of the substance to be photochemically excited include delayed fluorescent substances such as lanthanide complexes (e.g., europium complexes) (Hemmila, I. et al., Drug Discovery Today, 2, 373-381, 1997). Examples of the substance to be chemically excited include chemiluminescent substances such as luminol.

Examples of "energy" as used herein include photoenergy, thermoenergy, electromagnetic energy and chemical energy.

The term "energy-absorbing substance" refers to a substance in which energy absorption is intercepted by the hybridization of a probe with a complementary nucleic acid, and preferably to a photoenergy-absorbing substance. Examples of such a photoenergy-absorbing substance include those capable of causing intercalation in a double-stranded nucleic acid (intercalators) and those specifically bound to a double-stranded nucleic acid. Examples of the photo-absorbing intercalator include acridine, anthracene, pyrene and derivatives thereof. Further, examples of such a photo-absorbing substance selectively bound to a double-stranded nucleic acid include ethidium bromide and Hoechst 33258.

These energy-absorbing substances can be any substance that is capable of absorbing energy released from a labeling substance and releasing the absorbed energy either as light, for example fluorescence, or as heat.

In the present invention, the combination of a labeling substance and an energy-absorbing substance is not particularly limited as long as energy transfer from the labeling substance to the energy-absorbing substance occurs. Examples of the combination include fluorescein and pyrene; fluorescein and acridine; fluorescein and coumarin; and tetramethylrhodamine and pyrene.

A labeling substance and an energy-absorbing substance can be introduced as they are directly into a specified position of an oligonucleotide using an automatic synthesizer if their modified forms for nucleic acid synthesis are available. Further, if such reagents are not available, functional groups such as an amino group and thiol group are introduced by using a nucleic acid-synthesizing reagent to introduce such functional groups in the process of oligonucleotide synthesis, after which labeling can be carried out by using a labeling into which a functional group which reacts with these functional groups are introduced. Introduction of the labeling with these reagents into the oligonucleotide can be on such positions as the 5' terminus or 3' terminus, or phosphoric acid moiety, or further a base or sugar moiety on a specified position. Further, it can be introduced between phosphoric acid diester bonds (Goodchild, J., Bioconhugate Chemistry, 1, 165-187, 1990).

In the present invention, efficient energy transfer from a labeling substance to an energy-absorbing substance has to occur, and thus these two labels have to be introduced into such a position that energy transfer occurs efficiently depending on the combination of the labels. It is generally believed that the more closely the two labels are arranged, the better energy transfer efficiency can be. However, the arrangement has to be discussed according to the combination of the labels since there may be exceptions (Morrison L.E., Nonisotopic DNA Probe Techniques, 311-352, Academic Press Inc., 1992).

The arrangement of two labels can be controlled by changing either the introduction position in an oligonucleotide or the structure or the length of the spacer to link a nucleic acid and the labels. Accordingly, the labels have to be arranged so as to produce energy transfer from a labeling substance to an energy-absorbing substance, but at the same time not to intercept the energy-absorbing substance from being intercalated into or binding to the formed double-stranded chain. Further, upon introduction of a fluorescent substance into an oligonucleotide, quenching of fluorescence by nucleic acid nucleotides can occasionally be observed depending on oligonucleotide nucleotide sequences, which has to be taken into consideration in the present invention. However, this quenching phenomenon may occasionally be compromised by the double-stranded chain formation.

The probe according to the present invention can comprise a nucleic acid of 4 nucleotides or more, more preferably 8 nucleotides or more.

The term "nucleic acid" as used herein refers not only to DNA and RNA but also to those that form a double-stranded chain with a nucleic acid, such as modified nucleic acids and PNA (peptide nucleic acid). Needless to say, the formed double-stranded chain has a structure to which an energy-absorbing substance can bind.

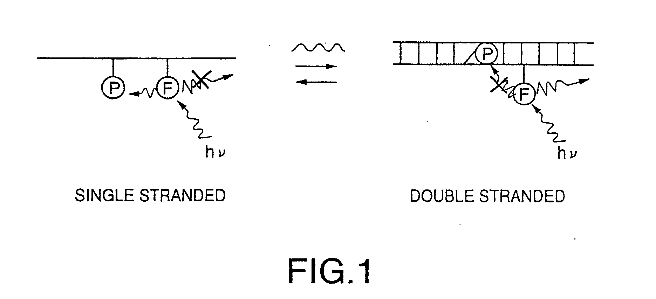

When a probe according to the present invention is completely complementary to a target nucleic acid, an energy-absorbing substance which is introduced into the probe, such as a photo-absorbing intercalator or photo-absorbing substance that binds specifically to a double-stranded nucleic acid, does interact with a double-stranded nucleic acid, resulting in no quenching of a labeling substance introduced into the probe (see Figure 1). On the other hand, when the probe does not hybridize or incompletely hybridizes with a sample, photo-absorbing intercalator or photo-absorbing substance that binds specifically to a double-stranded nucleic acid, which is introduced into the probe, does not interact with a double-stranded nucleic acid, resulting in quenching of a labeling substance introduced into the probe (see Figure 1). Thus, upon the hybridization with the nucleic acid sample, the presence of light released from the labeling substance indicates that the probe and the target nucleic acid are hybridized while the absence of light released from the labeling substance indicates that the probe and the target nucleic acid are not hybridized.

A probe according to the present invention can also detect a single base mismatch. Namely, when the probe and a sample do not match on a single base position, change in the structure of the resulting double-stranded nucleic acid occurs and an energy-absorbing substance introduced into the probe, such as a photo-absorbing intercalator or photo-absorbing substance that binds specifically to a double-stranded nucleic acid, does not interact with said double-stranded nucleic acid, resulting in quenching of a labeling substance introduced into the probe.

In the hybridization protection method (Nelson, N.C. et al., Nucleic Acids Res., 24, 4998-5003, 1988), it has been confirmed that volunerability to alkaline hydrolysis of an acridinium ester changes depending on whether a target and a probe are completely matched or mismatched with a single base, and thus the single base mutation is considered to be detected. Namely, acridine, an intercalator, is intercalated in different way depending on whether the double-stranded chain is completely matched or mismatched with a single base, and thus it is believed that acridine is probably intercalated in a completely matched double-stranded chain, but no intercalation occurs when a mismatch is present.

Thus, energy release from a labeling substance is not intercepted by an energy-absorbing substance when a complementary nucleic acid is present in a sample. Accordingly, the complementary nucleic acid in the sample can be detected by bringing a probe into contact with the sample and measuring energy release, such as fluorescence from the labeling substance.

More specifically, a probe of the present invention and a nucleic acid prepared from a sample are mixed and hybridized in a solution containing an appropriate buffer solution and the like, and then fluorescence or the like in the reaction solution is measured as it is and compared with a control to examine whether the sequence complementary to the probe is present in the sample.

Hybridization can be carried out by using an ordinary method and conditions (Keller, G.H. et al., DNA Probes, Stockton Press, 1993). Detection after hybridization can be carried out by using a detection device appropriate to the labeling substance used. For example, when the labeling substance is fluorescence, a fluorescence spectrophotometer or the like can be used. However, when a large number of samples are to be examined, detection can easily be carried out by using a fluorescence microplate reader or the like. Further, as mentioned below, when hybridization is carried out with a probe immobilized on a carrier, such as a gene chip, fluorescence on the carrier can be measured using a fluorescence scanner or the like. These measurements are generally carried out when an equilibration state is attained after hybridization. However, kinetics associated with a temperature change can be measured to observe more precise characteristics of the probe and sample nucleic acid (Howell, W.M. et al., Nature Biotechnology, 17, 87-88, 1999). Namely, a single base mismatch or the like can be detected more accurately by this method.

In the present invention, a sample nucleic acid can be either DNA or RNA, which can be an extract or crude product from any living organism. Further, when an extremely small amount of sample nucleic acid is used, the gene is amplified by a gene amplification method such as a PCR method, and then hybridization with a probe of the present invention can be carried out.

Further, the hybridization and measurement can be carried out continuously in a single vessel using an apparatus capable of changing the temperature equipped with a plurality of reaction vessels (ABI PRSM™ 7700, Perkin-Elmer), which can readily be subjected to fluorescence measurement.

The probe according to the present invention can be used not only in a solution for homogeneous hybridization but also in the state of being bound to a solid phase carrier. The probe according to the present invention which binds to a solid phase carrier can be prepared by synthesizing an oligonucleotide on the solid phase to be used for detection (Fodor, S.P.A. et al., Science, 251, 767-773, 1991) or by first synthesizing an oligonucleotide into which an appropriate functional group is introduced and then binding it to the solid phase for detection by utilizing the introduced functional group (Rasmussen, S.R., Anal. Biochem., 198, 138-142, 1991). Further, bonding between a ligand into which an oligonucleotide is introduced and a solid phase on which its receptor is immobilized can be utilized (Broude, N.E., Proc. Natl. Acad. Sci., 91, 3072-3076, 1994).

Examples of the solid phase carrier include a microtiter plate (Rasmussen, S.R., Anal. Biochem., 198, 138-142, 1991), a slide glass (Fodor, S.P.A. et al., Science, 251, 767-773, 1991), and an optical fiber tip (Ferguson, J.A., Nature Biotechnology, 14, 1681-1684, 1996).

Further, the solid phase carrier according to the present invention can be a gene chip. Gene chip technology is generally utilized in two cases, i.e., where the amount of RNA expression is mainly examined and where the gene mutation is examined (Lander, E.S., Nature Genetics supplement, 21, 3-4, 1999). The probe according to the present invention can be an efficient means in either case, but it can be highly effective particularly in the former case. In a conventional gene chip, an oligonucleotide, cDNA or the like is immobilized on a chip (Duggan, D.J. et al., Nature Genetics supplement, 21, 10-14, 1999; Lipshutz, R.J., Nature Genetics supplement, 21, 20-24, 1990), and a nucleic acid in a sample has to be labeled in order to detect the hybridization. Generally, in the detection with a gene chip, a labeling substance is introduced when a gene is amplified using a gene amplification method, and thus a quantitative measurement of RNA expression often fails because of the gene amplification. Furthermore, RNA is unstable and highly vulnerable to decomposition in various labeling reactions. Therefore, by immobilizing the probe according to the present invention on a chip, mRNA extracted from a living organism or the like can be detected by hybridization without labeling or any other processing. It is believed that by using such methods, gene expression analysis can be highly simplified, and an extremely large number of samples can easily be treated.

### EXAMPLE

An oligonucleotide was synthesized by a phosphoramidite method using an automatic nucleic acid synthesizer (DNA/RNA synthesizer Model 392, Perkin-Elmer). Fluorescein was introduced using fluorescein phosphoramidite (Glen Research, Cat. No: 10-1963). Pyrene was introduced by introducing an amino group using a Unilink™ Amino Modifier (Clontech, Cat. No: 5190-1), deblocking, and then reacting with 1-pyrenebutanoic acid succinimidyl ester (Molecular Probe, Cat. No: P-130). Nonlabeled oligonucleotides were purchased from Amersham Pharmacia Biotech.

### Example 1: Synthesis of oligonucleotides into which fluorescein and pyrene are introduced

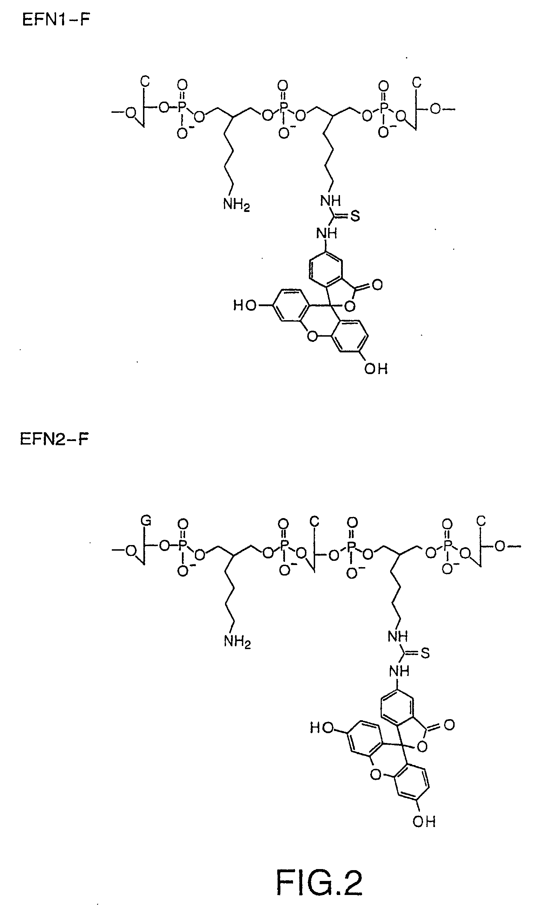

The following oligonucleotides into which an amino group and fluorescein were introduced were synthesized using an automatic synthesizer. F represents fluorescein. Embodied structures of the modified parts are shown in Figure 2. Further, fluorescein was confirmed by measuring fluorescent spectra (excitation wavelength: 494 nm, maximum fluorescent wavelength: 517 nm).

EFN1-F: 5′ GCAACAGGC(NH2)(F)CGACAACG (SEQ ID NO: 1)
EFN2-F: 5′ GCAACAGG(NH2)C(F)CGACAACG (SEQ ID NO: 1)
EFN3-F: 5′ ACGCACAAA(NH2)F)ACCAAGCA (SEQ ID NO: 2)
EFN4-F: 5′ ACGCACAA(NH2)A(F)ACCAAGCA (SEQ ID NO: 2)

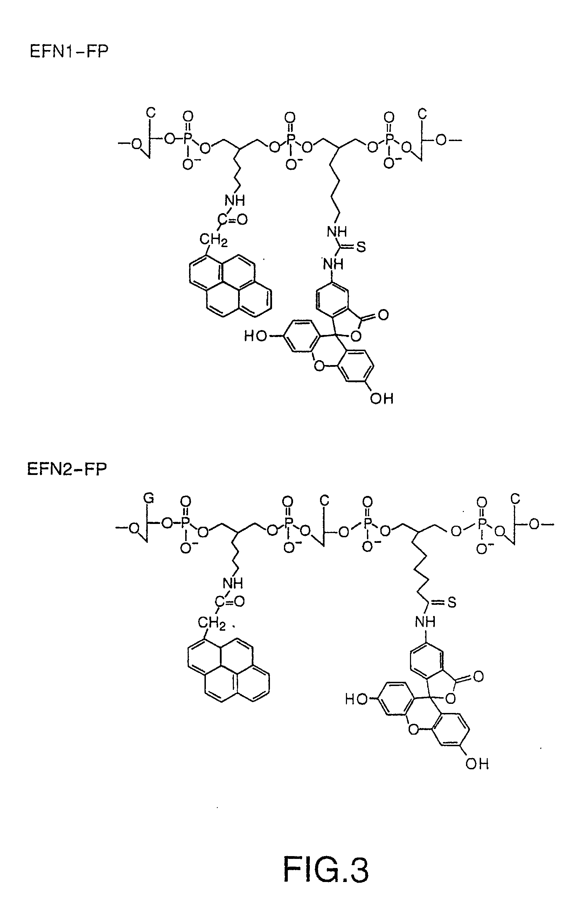

After deprotection, purification was carried out using denatured 20% polyacrylamide gel electrophoresis. To the purified oligonucleotide (80 µg) were added 1M NaHCO₃ (4 µl), a pyrene-active ester of DMF solution (20 µg/µl, 20 µl), and H₂O (16 µl), and reaction was carried out at 25°C for 14 hours. After reaction, excessive reagents were removed by gel filtration (Sephadex G-50, 50 mM TEAB buffer), and after concentration, purification was carried out by reverse-phase HPLC (µ-Bondapak C18, Waters). The resulting oligonucleotides are shown below. P represents pyrene, F represents fluorescein, and structures of the modified parts are shown in Figure 3. Introduction of pyrene was confirmed by measuring fluorescent spectra (excitation wavelength: 341 nm; maximum fluorescent wavelength: 383 nm, 400 nm).

EFN1-FP: 5′ GCAACAGGC (P) (F) CGACAACG (SEQ ID NO: 1)
EFN2-FP: 5′ GCAACAGG (P) C (F) CGACAACG (SEQ ID NO: 1)
EFN3-FP: 5′ ACGCACAAA (P) (F) ACCAAGCA (SEQ ID NO: 2)
EFN4-FP: 5′ ACGCACAA (P) A (F) ACCAAGCA (SEQ ID NO: 2)

### Example 2: Augmentation of fluorescent intensity by hybridization of fluorescein-pyrene-labeled oligonucleotides

The following non-labeled oligonucleotides complementary to the oligonucleotides having the abovementioned two kinds of nucleotide sequences were prepared.

EC1: 5′ CGTTGTCGGCCTGTTGC (SEQ ID NO: 3)
EC2: 5′ TGCTTGGTTTTGTGCGT (SEQ ID NO: 4)

The labeled oligonucleotides and non-labeled oligonucleotides were mixed with a buffer solution [50 mMTris-HCl (pH 8.0), 5 mM EDTA, 250 mM NaCl, 50 ng/µl carrier DNA] as shown below to prepare double-stranded chain solutions and control solutions. The mixed solutions were annealed from 94°C to 34°C for 5 hours.

**Table 1**

| No. | Labeled oligonucleotide (0.09 µg/µl) | Nonlabeled oligonucleotide (0.09 µg/µl) | Buffer solution | H₂O | Fluorescent intensity |
|---|---|---|---|---|---|
| 1 | EFN1-F: 10 µl | None | 20 µl | 70 µl | 256 |
| 2 | EFN1-F: 10 µl | EC1: 10 µl | 20 µl | 60 µl | 542 |
| 3 | EFN1-F: 10 µl | EC2: 10 µl | 20 µl | 60 µl | 282 |
| 4 | EFN2-F: 10 µl | None | 20 µl | 70 µl | 525 |
| 5 | EFN2-F: 10 µl | EC1: 10 µl | 20 µl | 60 µl | 823 |
| 6 | EFN2-F: 10 µl | EC2: 10 µl | 20 µl | 60 µl | 589 |
| 7 | EFN3-F: 10 µl | None | 20 µl | 70 µl | 1,137 |
| 8 | EFN3-F: 10 µl | EC1: 10 µl | 20 µl | 60 µl | 1,296 |
| 9 | EFN3-F: 10 µl | EC2: 10 µl | 20 µl | 60 µl | 1,095 |
| 10 | EFN4-F: 10 µl | None | 20 µl | 70 µl | 1,005 |
| 11 | EFN4-F: 10 µl | EC1: 10 µl | 20 µl | 60 µl | 1,203 |
| 12 | EFN4-F: 10 µl | EC2: 10 µl | 20 µl | 60 µl | 1,125 |
| 13 | EFN1-FP: 10 µl | None | 20 µl | 70 µl | 156 |
| 14 | EFN1-FP: 10 µl | EC1: 10 µl | 20 µl | 60 µl | 661 |
| 15 | EFN1-FP: 10 µl | EC2: 10 µl | 20 µl | 60 µl | 160 |
| 16 | EFN2-FP: 10 µl | None | 20 µl | 70 µl | 243 |
| 17 | EFN2-FP: 10 µl | EC1: 10 µl | 20 µl | 60 µl | 938 |
| 18 | EFN2-FP: 10 µl | EC2: 10 µl | 20 µl | 60 µl | 269 |
| 19 | EFN3-FP: 10 µl | None | 20 µl | 70 µl | 321 |
| 20 | EFN3-FP: 10 µl | EC1: 10 µl | 20 µl | 60 µl | 342 |
| 21 | EFN3-FP: 10 µl | EC2: 10 µl | 20 µl | 60 µl | 985 |
| 22 | EFN4-FP: 10 µl | None | 20 µl | 70 µl | 510 |
| 23 | EFN4-FP: 10 µl | EC1: 10 µl | 20 µl | 60 µl | 508 |
| 24 | EFN4-FP: 10 µl | EC2: 10 µl | 20 µl | 60 µl | 1,042 |

A measuring solution (10 mM Tris-HCl, 1 mM EDTA, 50 mM NaCl, 10 ng/µl carrier DNA; 420 µl) was added to the solution treated for annealing (30 µl), and then fluorescence was measured using Shimazu RF-5000 (Ex = 494 nm, Em = 518 nm). Samples with readings exceeding the scale were appropriately diluted for measurements, and the fluorescent intensity of each original solution was calculated by appropriate conversion. The resulting fluorescent intensities are shown in Table 1 and also in Graph 1.

Some samples into which pyrene was not introduced show increased fluorescent intensity due to the formation of a double-stranded chain (No. 4 and No. 5). This is because fluorescence of fluorescein introduced into an oligonucleotide, which had been quenched by the interaction with the oligonucleotide, was released by the double-stranded chain formation. Such a phenomenon occurs often depending on sequences, and cannot be observed in No.7 through No. 12. On the other hand, in samples into which pyrene was introduced, fluorescent intensity increased only when a double-stranded chain was formed (Nos. 14, 17, 21, and 24, among Nos. 13 through 24), which shows that this probe increases fluorescent intensity by hybridization.

## Claims

1. A probe comprising a nucleic acid carrying a labeling substance that releases energy and an energy-absorbing substance capable of absorbing the energy released from the labeling substance, wherein energy transfer from the labeling substance to the energy-absorbing substance is intercepted by the hybridization of the probe with a target nucleic acid.

2. The probe according to claim 1, wherein the energy is photo energy.

3. The probe according to claim 1 or 2, wherein the labeling substance is selected from the group consisting of a fluorescent substance, a delayed fluorescent substance, and a chemiluminescent substance.

4. The probe according to any one of claims 1 to 3, wherein the energy-absorbing substance is an intercalator or a substance which specifically binds to a double-stranded nucleic acid.

5. The probe according to claim 4, wherein the intercalator is selected from the group consisting of acridine, anthracene, pyrene, and derivatives thereof.

6. The probe according to claim 1 or 2, wherein the labeling substance is fluorescein, and the energy-absorbing substance is selected from the group consisting of pyrene, coumarin, and acridine.

7. A solid phase carrier for detecting a nucleic acid, on which the probe of any one of claims 1 to 6 is immobilized.

8. A method for detecting a nucleic acid comprising the steps of contacting the probe of any one of claims 1 to 6 with a nucleic acid sample and then measuring energy released from the labeling substance.

9. The method according to claim 8, wherein the presence of the energy released from the labeling substance indicates the hybridization of the probe with the target nucleic acid.
